# EUROPEAN PATENT APPLICATION

(11) **EP 4 417 115 A1**
(43) Date of publication of application: **21.08.2024**
(21) Application number: 23157241.3
(22) Date of filing: 17.02.2023
(51) Int. Cl.: A61B 5/0215

(54) **BLOOD PRESSURE SIGNAL PROCESSING**

(71) Applicant: Koninklijke Philips N.V., 5656 AG Eindhoven (NL)
(72) Inventor: FRANCK, Christoph Florian, Eindhoven (NL)
(74) Representative: Philips Intellectual Property & Standards

(57) **Abstract**

The invention provides a method for filtering an invasive blood pressure (IBP) measurement signal based on use of a low pass filter with a frequency response curve which is adapted to exhibit shelving behavior, whereby a higher frequency portion of the passband exhibits greater attenuation behavior than a typical low-pass filter. This has the effect of suppressing underdamping and catheter whip artefacts without eliminating signal components at frequencies in the higher frequency portion of the passband.

## Description

### FIELD OF THE INVENTION

The present invention relates to the field of invasive blood pressure measurement.

### BACKGROUND OF THE INVENTION

The present invention aims to provide a development in the field of invasive blood pressure measurement. In particular, the claimed method aims to provide the effect of improved accuracy of obtained invasive blood pressure measurements by means of a new method for signal processing aiming at removing or minimizing two common types of signal artefact.

Invasive blood pressure (IBP) refers to a clinical measurement in which a fluid-filled catheter is inserted into an artery of the patient. The catheter is connected to a system of fluid-filled tubing outside the patient's body that acts as a conductor for the intra-arterial pressure. The blood pressure inside the artery is measured by a pressure transducer connected to the tubing system. This measurement is invasive, but is faster, more direct and more accurate than known non-invasive methods for measuring blood pressure.

The IBP measurement is sensitive to the hydraulic behavior of the catheter and tubing system. Excessive tubing length or small air bubbles trapped in the tubing system can change the frequency response of the setup, leading to underdamping. This manifests as the recorded pressure wave overshooting the actual pressure in the blood vessel after sudden pressure changes, such as systole. This may typically therefore manifest as an overshoot in the magnitude of the peak pressure within a particular heart cycle.

The IBP measure is furthermore sensitive to movement of the catheter tip, which causes brief but large oscillations/fluctuations or spike-like artefacts in the recorded pressure wave. These can in some cases have an appearance which is similar to artefacts caused by underdamping, although caused by a different underlying mechanism. Both types of artefacts are well known and described in literature.

Both underdamping and catheter whip artefacts adversely affect the ability to interpret and analyze the pressure waveform, for example determining the systolic, diastolic and mean blood pressure. Especially systolic and mean blood pressure can be significantly overestimated when one or both artefacts are present. Pathological conditions may be masked by the artefacts and necessary treatment may be delayed or erroneously omitted. Conversely, the artefacts may lead to false positives in diagnosis, whereupon a patient may receive unnecessary and potentially harmful treatment.

Currently, underdamping and catheter whip artefacts are addressed by adaptation of the hardware including choosing a catheter and tubing system which is not excessively long, using stiff tubing with an appropriate diameter, and by flushing the tubing system to remove any tapped air bubbles. Hydraulic damping devices are also known which can be inserted into the tubing system to provide additional damping (e.g. the resonance over-shoot eliminator (R.O.S.E), from Medline Industries Inc.).

Digital filtering of the pressure waveform is also used. However, regular low-pass filters either fail to provide sufficient damping or have a cut off frequency which is too low, meaning that signal strength of the blood pressure is diminished, particularly for outlier cases, where components of the blood pressure can extend up to around 10-14 Hz. Indeed, applicable industry standards at the time of writing require any filter to have a cut-off frequency of at least 10 Hz.

For example, linear filters, implemented either as hydraulic dampers, analog circuitry or digital filters, may be used to attenuate artefacts caused by underdamping or catheter whip. However, applicable standards like IEC 60601-2-34 place restrictions on the frequency response of the recording system by requiring that the -3dB corner frequency is not lower than 10 Hz. Common low-pass filter types designed with this corner frequency will not provide sufficient attenuation when large artefacts are present.

### SUMMARY OF THE INVENTION

Thus, the inventors have recognized that there is a need to provide a filter which permits greater suppression of underdamping and catheter whip artefacts without completely cutting off signal frequency components within the frequency range in which these artefacts typically manifest.

To achieve thus, the inventors have further recognized that one solution is to provide e a filter which permits setting a cut-off frequency which is potentially significantly higher than a point at which at least partial attenuation would be applied. In this way, the underdamping and catheter whip artefacts which have characteristic frequencies which overlap with the required passband range of the standard low-pass filter can be at least partially suppressed.

To solve the problem, the inventors have turned to the remote technical field of audio signal processing. In this field, it is known to use so-called `shelving filters', or low pass filters with shelving behavior. A shelving filter is a low-pass filter which has an inflection point in the gain response part way across the passband, so that the passband effectively has two frequency sub-ranges, one with a relatively high gain, and one with a relatively lower gain (i.e. greater attenuation). This provides a filter which acts substantially like a low pass filter across its lower frequency range, while providing partial attenuation across its higher frequency range.

The invention is defined by the claims.

According to examples in accordance with an aspect of the invention, there is provided a processing device for use in an apparatus for invasively measuring a blood pressure inside a portion of the cardiovascular system, the processing device comprising: receive circuitry adapted to receive a blood pressure measurement signal indicative of a pressure inside a portion of the cardiovascular system, and an electronic filter arranged to filter the blood pressure measurement signal. The filter may be a low-pass filter, wherein a passband of the filter has a lower frequency range with a lower boundary of 0 Hz, and wherein the passband of the filter further has an upper frequency range spanning from an upper boundary of the lower frequency range to a cut-off frequency of the filter. Across the lower frequency range of the passband, a gain of the filter, G(f), is greater than or equal to about -0.5 dB. Across the higher frequency range of the passband, the gain of the filter, G(f), is between about -0.5dB and -3dB. An average gain of the filter in the higher frequency range is less than about -1.7 dB, and/or the average gain of the filter across a frequency range spanning the whole of both the lower frequency range and the higher frequency range is less than about -1.2dB.

For avoidance of doubt, the cut-off frequency is defined by the frequency coinciding with a filter gain of -3dB.

Thus, there is provided a low -pass filter with shelving behavior. This filter offers partial attenuation of signal frequency components across a higher frequency portion of the passband without a need to set the cut-off frequency of the filter significantly lower.

As will be demonstrated later, this has the effect of significantly suppressing signal artefacts associated with underdamping and catheter whip (namely overshoot of the peak signal amplitude and small amplitude fluctuations in the signal mid-cycle), but without fully eliminating true blood pressure signal components which may be present within the higher frequency range of the passband.

The use of filters with shelving-type behavior is not known in the present technical field. In the current state of the art for the field of invasive blood pressure measurement, it seen as optimal to apply low pass filters with average gain as close as possible to 0 dB. The insight of the inventors of the present invention is that low-pass filter behavior can be combined with shelving filter behavior in order to provide an optimized balance between passband width and artifact suppression.

The electronic filter can be understood as a low pass filter with a gain response which is modified to provide the above-described characteristics. For example, the electronic filter may be described as a low pass filter with the gain response adapted to exhibit shelving filter behavior across a higher frequency portion of the passband. By low-pass filter is meant for example a filter with a frequency-gain response which starts at 0 dB at frequency 0 Hz and then progresses according to a frequency-gain curve to a cut-off frequency (at -3dB). For a typical low-pass filter, the frequency-gain curve would remain as close to 0dB as possible for the majority of the pass-band, so that the response as closely as possible approximates a rectangular function. Embodiments of the present invention seek to modify the response so as to deliberately introduce a region of the passband at higher frequencies that have lower gain response.

It will be understood from this that there are in fact different ways to characterize or describe the required filter properties. One way to characterize the filter is a low pass filter wherein the average gain across the whole passband is less than about -1.2dB. This distinguishes the filter from typical low-pass filters where average gain would typically be higher than this, much closer to the ideal of 0dB. Since all low-pass filters start as 0dB at 0Hz, this condition necessarily implies a reduced gain response across a higher frequency range, which is the technical aim for suppressing the discussed artefacts. Another way to characterize the filter is to define the filter gain response as exhibiting a higher frequency range portion and lower frequency range portion, the boundary between the two defined for example by a point at which the gain is -0.5dB, and wherein an average gain across the higher frequency range is below about -1.7 dB. Again, for typical low-pass filters the average gain across the higher frequency range would normally be much higher (closer to zero). Thus, an average gain of -1.7 dB distinguishes a filter with shelving behavior from a regular low-pass filter. This characterization again captures the requirement for partial attenuation across a higher frequency section of the passband.

It is noted that the reference point for the defined gain may for example be DC or may be a frequency in the lower third of the lower frequency range. For example for a lower frequency range of 0Hz to 3Hz the reference point for the gain/attenuation measurements could be between 0 and 1 Hz.

In some embodiments, the lower boundary of the lower frequency range is 0 Hz and an upper boundary of the lower frequency range may be no more than 4Hz.

In some embodiments, the upper boundary of the lower frequency range is no more than 3 Hz.

In some embodiments, the upper boundary of the lower frequency range is no more than 2 Hz.

In some embodiments, the upper boundary of the lower frequency range is no less than 1 Hz.

In some embodiments, the higher frequency range spans from the upper boundary of the lower frequency range to the cut-off frequency of the filter, wherein the cut-off frequency of the filter is at least 10 Hz, preferably at least 12 Hz, preferably at least 13 Hz.

The cutoff frequency, corner frequency, or break frequency is the boundary in the system's frequency response at which energy flowing through the system begins to be reduced (attenuated or reflected) rather than passing through. It is, by definition, the frequency at the -3dB point of the filter response.

Current applicable standards, such as IEC 60601-2-34, place restrictions on the frequency response of the recording system by requiring that the -3dB corner frequency is not lower than 10 Hz.

The filter can be implemented as a digital filter or an analog filter, more preferably a digital filter.

The invasive blood pressure measurement signal is preferably a digitally sampled signal.

In some embodiments, the device is adapted to be selectively operable in a plurality of modes. The device may be configured such that, in at least one mode, the electronic filter as defined in any of the examples of embodiments described in this disclosure (i.e. having the shelving behavior) is applied to the measured invasive blood pressure signal. The device may be configured such that, in at least a further mode, a different, low-pass filter is applied.

The different low pass filter applied in the at least further mode may have an average gain across the whole of the passband of greater than -1.2dB, and/or an average gain across the higher frequency range of the passband (as defined according to the previously referenced definition) of greater than -1.7dB. The different low pass filter may thus be a standard low-pass filter without the shelving behavior.

The plurality of modes may include at least two modes in each of which a respective electronic filter in accordance with the invention is applied to the measured invasive blood pressure measurement signal, i.e. having the shelving behavior. The respective filters applied in the two modes may differ in respect of a percentage of a total frequency width of the passband for which the gain response of the filter remains above about -0.5dB. In this way these two modes both provide the artefact suppression benefits of the shelving filter response, but are differently tuned as to the frequency point at which the gain falls to a point where significant attenuation begins to be applied. In other words, the two filters differ in respect of the portion of the passband over which the partial attenuation is applied. By way of example, the plurality of filters may include a first filter for which the gain response falls below -0.5dB at around 2Hz, a further filter for which the gain response falls below -0.5dB at around 3Hz and a further filter for which the filter falls below -0.5dB at around 4Hz.

Preferably at least a further mode is provided in which a normal low-pass filter is applied, i.e. having an average gain across the whole of the passband of greater than -1.2dB, and/or an average gain across the higher frequency range of the passband (as defined according to the previously referenced definition) of greater than -1.7dB. In some embodiments, a plurality of such low-pass filters may be provided, each having a different respective cut-off frequency, for example one filter with 12Hz cut off frequency and another filter with 40 Hz cut-off frequency.

In some embodiments, the device may include a user control permitting a user to select between the plurality of modes.

In some embodiments, the device includes a pressure calculation module for processing the filtered measurement signal to derive internal blood pressure measurement values.

Another aspect of the invention is a blood pressure measurement system for measuring an internal pressure inside a portion of the cardiovascular system, the system comprising: an invasive blood pressure measurement apparatus, for example comprising a pressure measurement catheter for insertion inside a portion of the cardiovascular system for measuring a pressure signal; and a processing device in accordance with any example or embodiment detailed in this disclosure.

In some embodiments, the system may further include a user interface for displaying calculated blood pressure measurement values.

Another aspect of the invention is an electronic filter for filtering an invasive blood pressure signal indicative of a pressure inside a portion of the cardiovascular system, the signal received from an internal measurement catheter. The filter is a low pass filter, wherein a passband of the filter has a lower frequency range with a lower boundary of 0 Hz, and wherein the passband of the filter further has an upper frequency range spanning from an upper boundary of the lower frequency range to a cut-off frequency of the filter. Across the lower frequency range of the passband, a gain of the filter, G(f), is greater than or equal to about -0.5 dB. Across the higher frequency range of the passband, the gain of the filter, G(f), is between about -0.5dB and -3dB. An average gain of the filter in the higher frequency range is less than about -1.7 dB, and/or the average gain of the filter across a frequency range spanning both the lower frequency range and the higher frequency range is less than about -1.3 or -1.2 dB.

Another aspect of the invention is a method comprising: receiving from an invasive blood pressure measurement apparatus a blood pressure signal indicative of a pressure inside a portion of the cardiovascular system, and processing the blood pressure signal with an electronic filter. The electronic filter is a low pass filter, wherein a passband of the filter has a lower frequency range with a lower boundary of 0 Hz, and wherein the passband of the filter further has an upper frequency range spanning from an upper boundary of the lower frequency range to a cut-off frequency of the filter. Across the lower frequency range of the passband, a gain of the filter, G(f), is greater than or equal to about -0.5 dB. Across the higher frequency range of the passband, the gain of the filter, G(f), is between about -0.5dB and -3dB. An average gain of the filter in the higher frequency range is less than about -1.7 dB, and/or the average gain of the filter across a frequency range spanning both the lower frequency range and the higher frequency range is less than about -1.2dB.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates the frequency response curve of a filter in accordance with one or more embodiments of the invention as compared against the response curves of standard low-pass filters;
Fig. 2 shows a circuit diagram of a suitable topology of one example analog filter;
Fig. 3 illustrates the performance of a filter in accordance with one or more embodiments by showing a blood pressure signal after filtering by a filter according to an embodiment of the invention as compared with the blood pressure signal after filtering by standard low-pass filters;
Fig. 4 is a block diagram of an example processing device and system in accordance with one or more embodiments of the invention; and
Fig. 5 outlines steps of an example method in accordance with one or more embodiments of the invention.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a method for filtering an invasive blood pressure (IBP) measurement signal based on use of a low pass filter with a frequency response curve which is adapted to exhibit shelving behavior, whereby a higher frequency portion of the passband exhibits a reduced gain response than a typical low-pass filter. This has the effect of suppressing underdamping and catheter whip artefacts without eliminating signal components at frequencies in the higher frequency portion of the passband.

Embodiments of the invention are intended to improve the quality of an IBP waveform recorded by an IBP recording system in the presence of underdamping or catheter whip artefacts. When implemented in practice, the proposed signal processing method may facilitate improved accuracy of invasive blood pressure measurement.

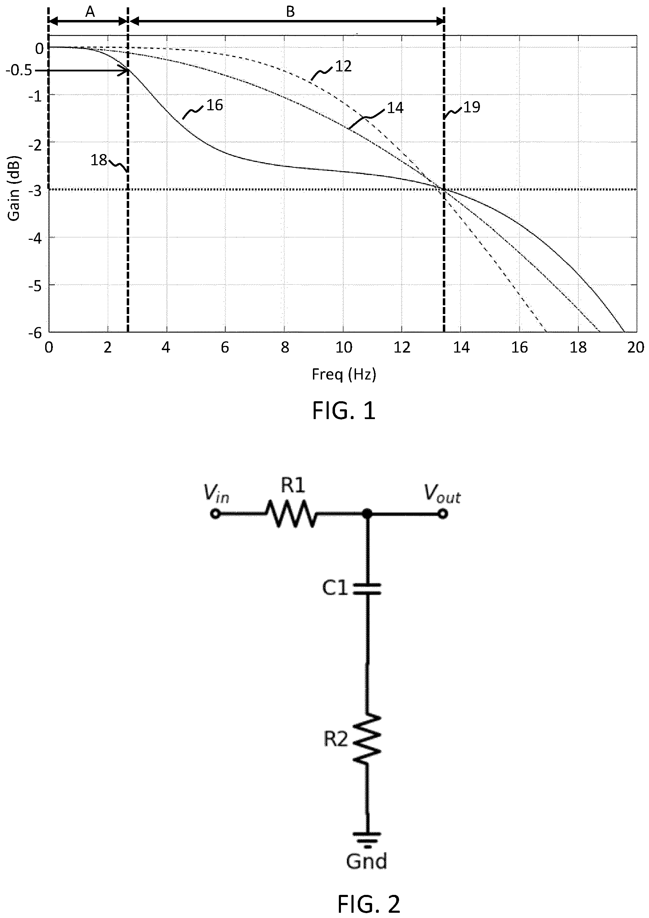

The proposed concept in accordance with one or more embodiments is illustrated by Fig. 1.

Fig. 1 shows the amplitude response curve for each of a first 12 and second 14 standard low pass filter, as well as the amplitude response curve 16 of a filter in accordance with one or more embodiments of the present invention, having shelving behavior. All three filters have approximately the same -3dB cut-off frequency (sometimes referred to as `corner frequency'). Thus, the frequency width of the passband of all three filters is approximately the same. However, it will be apparent that the gain response curve 16 of the filter according to an embodiment of the present invention exhibits stronger attenuation of frequencies within an upper frequency portion of the passband.

In the illustrated example, the higher frequency range portion (B) of the passband spans between about 2.5 Hz and 13.5 Hz.

There are different particular ways in which the required characteristics of the filter may be defined. The following represents at least one way of defining at least one set of embodiments of the invention.

According to an aspect of the invention, there is provided an electronic filter for filtering an invasive blood pressure signal indicative of a pressure inside a portion of the cardiovascular system, the signal received from a blood pressure measurement apparatus which may for example comprise an invasive measurement catheter.

With reference to Fig. 1, the filter is a low pass filter, wherein a passband of the filter has a lower frequency range (A) with a lower boundary of 0 Hz, and wherein the passband of the filter further has an upper frequency range (B) spanning from an upper boundary 18 of the lower frequency range to a cut-off frequency of the filter.

The lower frequency range (A) of the passband is defined as a range across which a gain of the filter, G(f), is everywhere greater than or equal to about -0.5 dB. The higher frequency range of the passband is defined as a frequency range across which the gain of the filter, G(f), is everywhere between about -0.5dB and -3dB (the latter being the cut-off frequency of the filter).

The filter is characterized in that an average gain of the filter in the higher frequency range (A) is less than (about) -1.7 dB, and/or the average gain of the filter across a frequency range spanning both the lower frequency range and the higher frequency range (i.e. the whole passband) is less than (about) -1.3dB or -1.2 dB.

Additionally or alternatively the filter is characterized in that a % proportion of the total passband frequency width within which the gain remains above -0.5dB is less than about 33% (i.e. less than about 1/3).

The average gain across a particular frequency range, Δf, may be computed based on the integral of the gain over the range, divided by the frequency width, Δf, of the frequency range. Alternatively, the average gain across a certain frequency range can be computed based on a sum of discrete gain sample points spanning the relevant frequency range, divided by the number of sample points (i.e. the arithmetic average).

As defined, this describes a family of filters that provide more attenuation of underdamping and catheter whip artefacts than a regular low-pass filter having the same cut-off frequency. Accordingly, greater suppression of the artefacts can be achieved without a need to change the cut-off frequency. This is beneficial since the blood pressure signal can in certain circumstances include frequency components which overlap with the typical frequency range of overdamping and catheter whip artefact-causing events. Thus, artefacts can be reduced with minimal loss of signal. In addition, this also advantageously allows for the filter to suppress the artefacts whilst remaining compliant with currently applicable standards for invasive pressure monitoring which, at the time of writing, require a cut-off frequency of at least 10Hz.

The defined family of filters thus combine shelving behavior and low-pass behavior in order to have significant attenuation in a section of the nominal passband of the filter (e.g. between -0.5 dB and -3 dB).

The filter may be provided as a digital filter or an analog filter.

To create a digital filter with the defined passband characteristics, design equations for shelving-type filters can be found in Chapter 5 of the book "Digital Audio Signal Processing, Second Edition" by Udo Zölzer, published by Wiley, August 2008.
Suitable design equations can also be found in the following article: Bristow-Johnson, Robert. "Cookbook formulae for audio equalizer biquad filter coefficients", available for download as of 14 February 2023 at the web address "https://webaudio.github.io/Audio-EQ-Cookbook/audio-eq-cookbook.html".

To create an analog filter with the defined passband characteristics, Fig. 2 illustrates a circuit diagram of a sample filter topology which could be used to provide a passive first-order analog shelving filter that attenuates high frequencies. It would be within the competence of a skilled person in this field to tune the filter characteristics to obtain a desired shelving cut-off.

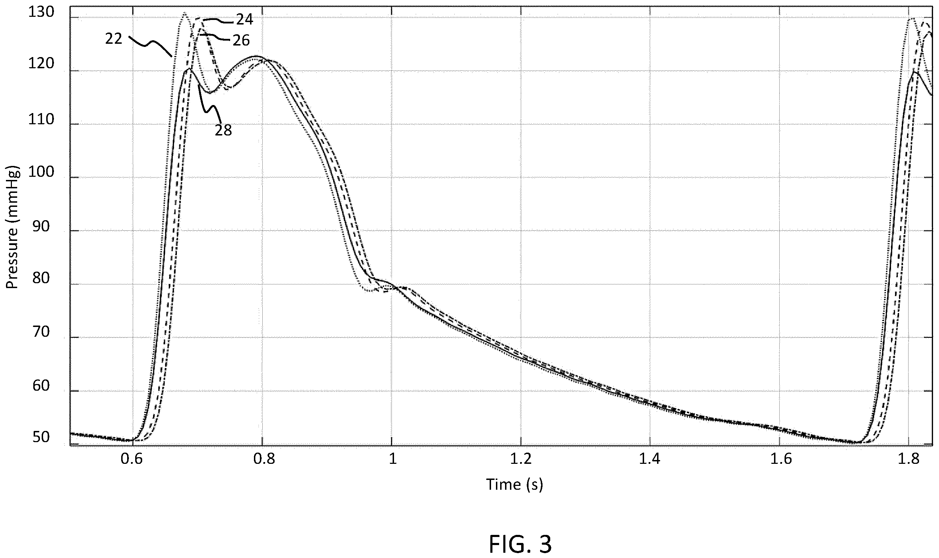

The artefact suppression effect of a filter in accordance with the invention is illustrated by Fig. 3. This illustrates a recorded invasive blood pressure measurement signal (y-axis; [mmHg]) as a function of time (x-axis; [s]) for each of four different cases as follows. Line 22 corresponds to a blood pressure measurement signal for an unfiltered signal subject to a catheter whip artefact. The catheter whip artefact manifests as an overshoot in the peak amplitude of the signal. Line 24 corresponds to the same blood pressure measurement signal but filtered using a standard lowpass filter having the frequency response of curve 12 in Fig. 1. Line 26 corresponds to the same blood pressure measurement signal but filtered using a standard lowpass filter having the frequency response of curve 14 in Fig. 1. Finally, line 28 corresponds to a same blood pressure measurement signal but filtered using a filter in accordance with an embodiment of the present invention, having the shelving behavior shown in frequency response curve 16 of Fig. 1. It can be seen that, compared to the unfiltered signal 22, and the signals 24, 26 filtered by the two standard low-pass filters, the signal 28 processed with a filter according to an embodiment of the present invention shows a markedly reduced artefact presence.

Thus, in summary, embodiments of the present invention propose a low-pass filter for an IBP recording system that exhibits shelving behavior. Shelf filters or Shelving filters differ from standard passband/stopband filters by having a gain greater than zero (on a linear scale) for all frequencies. A high shelving filter, for example, will have a gain of approximately one for low frequencies, and either an attenuation or an amplification of high frequencies.

Shelving filters are usually encountered in audio applications but find little use in other areas, as low-pass filters are typically expected to attenuate components in the passband as little as possible, and to attenuate components in the stopband as strongly as possible.

It is the recognition of inventors of the present invention that shelving filters may be used in the context of IBP measurement to attenuate underdamping or catheter whip artefacts while reproducing the pressure waveform faithfully enough so that systolic, diastolic and mean pressure measurement derived from the signal are not significantly affected. Additionally, the frequency response of the IBP recorder is compliant with the requirements imposed by the applicable standards.

Thus, the family of filters in accordance with the invention provide more attenuation of underdamping and catheter whip artefacts than regular low-pass filters, and at the same time avoid cutting off completely signal frequency components in the upper frequency portion of the passband.

The stopband of the filter begins, by definition, at the frequency where the gain reaches - 3 dB. The present invention places no constraints on the behavior of the filter in the stopband, other than the gain being less than -3dB.

With reference to Fig 1, the lower boundary of the lower frequency range (A) is 0 Hz. In some embodiments, the upper boundary 18 of the lower frequency range (A) may be no more than 4Hz. In some embodiments, the upper boundary 18 of the lower frequency range (A) is no more than 3 Hz. In the illustrated example, it is at around 2.5 Hz. In further embodiments, the upper boundary of the lower frequency range may be no more than 2 Hz. In some embodiments, the upper boundary of the lower frequency range may be no less than 1 Hz.

In some embodiments, the higher frequency range (B) may span from the upper boundary 18 of the lower frequency (A) range to the cut-off frequency 19 of the filter, wherein the cut-off frequency of the filter is at least 10 Hz, preferably at least 12 Hz, preferably at least 13 Hz. In the illustrated example of Fig. 1, the cut-off frequency is about 13.5 Hz.

The filter may be implemented in practice as part of a device or system for acquiring invasive blood pressure measurements.

Accordingly, according to an aspect of the device there is provided a processing device for use in an apparatus for invasively measuring a blood pressure inside a portion of the cardiovascular system.

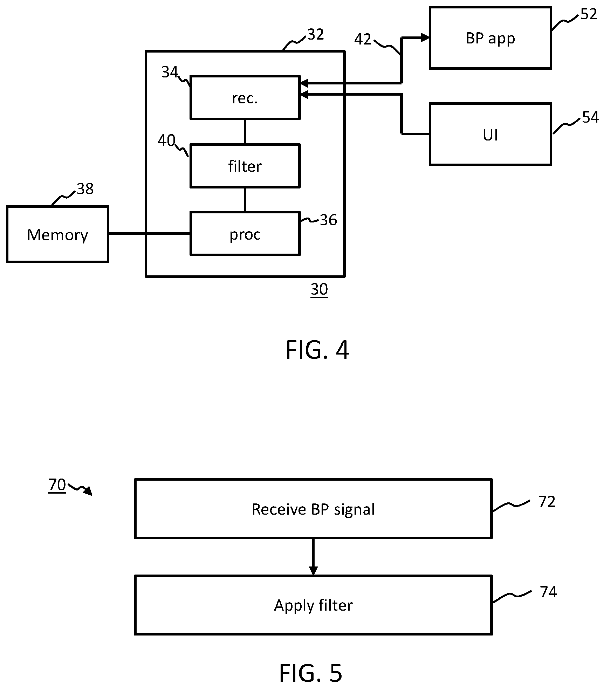

To aid understanding, Fig. 4 presents a schematic representation of an example processing device 32 in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only subset of them.

The processing device 32 may comprise receive circuitry ("rec") 34 adapted to receive an invasive blood pressure measurement signal 42 indicative of a pressure inside a portion of the cardiovascular system. The blood pressure measurement signal may be received from an invasive blood pressure measurement apparatus ("BP app") 52. The blood pressure measurement apparatus may for example comprise a fluid-containing catheter for insertion inside a portion of the cardiovascular system for measuring a pressure signal.

The processing device 32 further comprises an electronic filter 40 arranged to filter the received blood pressure measurement signal. The electronic filter may have the form in accordance with any of the embodiments described in this document or in accordance with any claim of this application.

The processing device may further comprise one or more processors 36 configured for performing one or more functions in the context of the processing device 32. In some embodiments, the one or more processors are arranged to receive a filtered measurement signal output from the electronic filter 40. In some embodiments, the one or more processors may include a pressure calculation module for processing the filtered measurement signal to derive internal blood pressure measurement values. This module may be a software module executed by the one or more processors or may be a dedicated hardware module comprised among the one or more processors.

In some embodiments, the one or more processors may additionally or alternatively be adapted to perform a control function within the processing device 32. In some embodiments, the system 30 may further comprise a memory 38 for storing computer program code (i.e. computer-executable code) which is configured for causing the one or more processors 36 of the processing unit 32 to perform a control method. For example, the method may comprise receiving from an invasive blood pressure measurement apparatus 52 (e.g. comprising a measurement catheter) an internal blood pressure signal indicative of a pressure inside a portion of the cardiovascular system, and controlling processing of the internal blood pressure signal with the electronic filter 40.

In some embodiments, the system 30 may further include a user interface ("UI") 54, for example for displaying calculated blood pressure measurement values.

Another aspect of the invention may provide a method. Fig. 5 outlines steps of one example method in accordance with at least one set of embodiments of the invention.

The method 70 comprises: receiving 72 from an invasive blood pressure measurement apparatus a blood pressure signal indicative of a pressure inside a portion of the cardiovascular system. The method further comprises processing 74 the blood pressure signal with an electronic filter. The electronic filter may be in accordance with any of the examples or embodiments described in this document or in accordance with any claim of this application.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

In some embodiments, the device may permit selectable configuration in one of a plurality of different available modes, wherein in each mode a different filter is implemented to filter the blood pressure measurement signal. The different filters may include a plurality of filters having the shelving behavior described above, but with different response function shapes and/or characteristics. The different filters may have different cut-off frequencies in some examples. The different filters may differ in the average gain across the higher frequency portion, accordingly, providing different levels of attenuation of the higher frequency components within the passband. In preferred embodiments, at least one of the available filters among the selection may include a standard lowpass filter without the shelving behavior so that the user has the option to filter the measurement signal in the way in which it is normally filtered in the state-of-the-art.

In preferred embodiments, there may be included among the plurality of available filters at least two different filters having the shelving behavior, but wherein the proportion of the passband across which partial attenuation is applied is different. For example, the different filters may include filters which differ in terms of the frequency point at which the gain of the filter response drops below about -0.5dB. For example, a useful set of filters for practical purposes might be a set of filters where this -0.5dB point occurs at between 2-3 Hz, between 3-4 Hz and between 4-5 Hz respectively (and wherein the point is different for each of the filters).

The different filters may be implemented as a separate physical modules within the device and wherein the device is configured to selectively connect in a chosen one of the filters in accordance with the selected setting. The setting may be selectable by a user for example using a user interface. Of course, the switching in can be done digitally. In some examples, the filters may be digital filters in which case each of them can be implemented through software code and switching between different filters may comprise simply switching between different software modules corresponding to each different selectable filter.

The device may include a user control permitting a user to select between the plurality of modes.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processing device (32) for use with an apparatus for invasively measuring a blood pressure inside a portion of the cardiovascular system, the processing device comprising:
receive circuitry (34) adapted to receive a blood pressure measurement signal indicative of a pressure inside a portion of the cardiovascular system, and
an electronic filter (40) arranged to filter the blood pressure measurement signal,
wherein the electronic filter is a low pass filter;
wherein a passband of the filter has a lower frequency range with a lower boundary of 0 Hz, and wherein the passband of the filter further has an upper frequency range spanning from an upper boundary of the lower frequency range to a cut-off frequency of the filter;
wherein, across the lower frequency range of the passband, a gain of the filter, G(f), is greater than or equal to about -0.5 dB;
wherein, across the higher frequency range of the passband, the gain of the filter, G(f), is between about -0.5dB and -3dB, and
wherein an average gain of the filter in the higher frequency range is less than about -1.7 dB, and/or the average gain of the filter across a frequency range spanning the whole of both the lower frequency range and the higher frequency range is less than about -1.2dB.

2. The device of claim 1, wherein the lower boundary of the lower frequency range is 0 Hz and wherein an upper boundary of the lower frequency range is no more than 4Hz.

3. The device of claim 1 or 2, wherein the upper boundary of the lower frequency range is no more than 3 Hz.

4. The device of claim 1 or 2, wherein the upper boundary of the lower frequency range is no more than 2 Hz.

5. The device of any of claims 1-4, wherein the upper boundary of the lower frequency range is no less than 1 Hz.

6. The device of any of claims 1-5, wherein the higher frequency range spans from the upper boundary of the lower frequency range to the cut-off frequency of the filter, wherein the cut-off frequency of the filter is at least 10 Hz, preferably at least 12 Hz, preferably at least 13 Hz.

7. The device of any of claims 1-6,
wherein the device is adapted to be selectively operable in a plurality of modes;
wherein, in at least one mode, the electronic filter as defined in any preceding claim is applied to the measured invasive blood pressure signal; and
wherein, in at least a further mode, a different, low-pass filter is applied.

8. The device of claim 7, wherein the plurality of modes includes at least two modes in each of which a respective electronic filter in accordance with any of claims 1-6 is applied to the measured invasive blood pressure measurement signal, and wherein the respective filters applied in the two modes differ in respect of a percentage of a total frequency width of the passband for which the gain response of the filter remains above about -0.5dB.

9. The device of claim 8, wherein the device includes a user control permitting a user to select between the plurality of modes.

10. The device of any of claims 1-9, wherein the processing device includes a pressure calculation module for processing the filtered measurement signal to derive internal blood pressure measurement values.

11. A blood pressure measurement system for measuring an internal pressure inside a portion of the cardiovascular system, the system comprising:
an invasive blood pressure measurement apparatus for measuring a pressure signal; and
a processing device as claimed in any preceding claim.

12. The device of claim 11, wherein the system includes a user interface for displaying calculated blood pressure measurement values.

13. An electronic filter for filtering an invasive blood pressure signal indicative of a pressure inside a portion of the cardiovascular system, the signal received from an invasive blood pressure measurement apparatus,
wherein the filter is a low pass filter;
wherein a passband of the filter has a lower frequency range with a lower boundary of 0 Hz, and wherein the passband of the filter further has an upper frequency range spanning from an upper boundary of the lower frequency range to a cut-off frequency of the filter;
wherein, across the lower frequency range of the passband, a gain of the filter, G(f), is greater than or equal to about -0.5 dB;
wherein, across the higher frequency range of the passband, the gain of the filter, G(f), is between about -0.5dB and -3dB, and
wherein an average gain of the filter in the higher frequency range is less than about -1.7 dB, and/or the average gain of the filter across a frequency range spanning both the lower frequency range and the higher frequency range is less than about -1.3 dB or -1.2 dB.

14. A method (70) comprising:
receiving (72) from an invasive blood pressure measurement apparatus a blood pressure signal indicative of a pressure inside a portion of the cardiovascular system, and
processing (74) the blood pressure signal with an electronic filter,
wherein the electronic filter is a low pass filter;
wherein a passband of the filter has a lower frequency range with a lower boundary of 0 Hz, and wherein the passband of the filter further has an upper frequency range spanning from an upper boundary of the lower frequency range to a cut-off frequency of the filter;
wherein, across the lower frequency range of the passband, a gain of the filter, G(f), is greater than or equal to about -0.5 dB;
wherein, across the higher frequency range of the passband, the gain of the filter, G(f), is between about -0.5dB and -3dB, and
wherein an average gain of the filter in the higher frequency range is less than about -1.7 dB, and/or the average gain of the filter across a frequency range spanning both the lower frequency range and the higher frequency range is less than about -1.2dB.
